# EUROPEAN PATENT APPLICATION

(11) **EP 4 850 607 A1**
(43) Date of publication of application: **09.12.2026**
(21) Application number: 25180618.8
(22) Date of filing: 04.06.2025
(51) Int. Cl.: A61B 6/03, A61B 6/00, A61B 6/50

(54) **SUBTRACTION-FREE, SERIAL PET PERFUSION IMAGING WITH REPEAT ADMINISTRATION OF A RADIOISOTOPE**

(71) Applicant: MedTrace Pharma A/S, 2970 Hørsholm (DK)
(72) Inventor: Harms, Hans, 2970 Hørsholm (DK); Lubberink, Mark, 2970 Hørsholm (DK)
(74) Representative: AWA Denmark A/S

(57) **Abstract**

The present invention relates to a non-invasive method for efficient multiscan PET diagnostics comprising dual administration of a radioisotope, and further comprises a method of recovering individual tracer signals from multi-tracer PET data.

## Description

### FIELD OF THE INVENTION

The present invention relates to a non-invasive method for efficient PET diagnostics comprising repeated administration of a short-lived radioisotope.

### BACKGROUND OF THE INVENTION

Many disease processes are defined by a lack of a physiological response to a trigger. These diseases typically relate to insufficient increase in blood flow, leading to tissue ischemia, and examples are coronary artery disease (CAD) or cerebral ischemia. To fully characterize these diseases, blood flow has to be measured at baseline as well as after maximal vasodilation is induced, or another physiological trigger is provided.

Positron Emission Tomography (PET) is an imaging modality in which a radiopharmaceutical is injected and tracked in a patient. The radiopharmaceutical allows the physician to visualize a (patho)physiological process, such as glucose consumption, blood flow, sympathetic innervation, and the presence of amyloid plaques. The radiopharmaceuticals are labeled with a radioisotope that decays with a certain half-life (t_{half}), and the distribution of these decays can be visualized by a PET scanner. The half-lives of PET radiopharmaceuticals range from several minutes (⁸²Rb, ¹⁵O) to several hours (¹⁸F) to days (⁸⁹Zr). Two commonly used radiopharmaceuticals for blood flow imaging are ¹³N-NH₃ (t_{half} = 9.96 minutes) and ¹⁸F-flurpiridaz (t_{half} = 110 minutes).

In some instances, a patient needs to receive multiple injections of a radiopharmaceutical, and scans associated with each injection to enable a correct diagnosis. Subsequent injections may be of the same radiopharmaceutical tracer as the first, or they may be different. The common practice is to let sufficient time elapse prior to each injection to allow the earlier introduced radiopharmaceutical to either flush from the patient or vanish through radioactive decay. As a general rule, significant activity can be measured for at least 8 half-lives, which means that clinical protocols with multiple radiopharmaceuticals are lengthy, as the time between consecutive scans has to be at least these 8 half-lives. This decreases throughput of patients and is inconvenient for patients in clinical applications. It further makes more personalized imaging with PET complicated, as it requires significant time from the patient, including possibly multiple trips to the hospital in a short amount of time. It is also a significant waste of resources for the hospital in terms of use of the PET scanners as well as the time of the technicians, physicians, and support staff.

One way of addressing this issue is by using a background correction method. Here, typically, the first injection is of a lower dose and the second injection is performed while there still is significant residual activity of the first injection present in the images. Prior to the second injection, the subject is imaged for 1-2 minutes. Then, the injection starts and the activity measured prior to injection is subtracted from all successive images.

This approach has already been implemented in commercial software packages (e.g. Invia's 4DM https://www.inviasolutions.com/packages), and can be represented as $\left({C}_{tissue}\left(t\right)-{C}_{tissue}\left({t}_{0}\right)\right) = {K}_{1} ⋅ {C}_{blood} \left(t\right) ⊗ {e}^{- {k}_{2} ⋅ t} + {V}_{blood} ⋅ {C}_{blood} \left(t\right)$

However, the subtraction method can be unstable, as it assumes that the empty frame is representative of the activity during the entire scan. This assumption is violated in cases of patient motion, or when the activity is not stable but increasing or decreasing. In addition, when the second scan is performed after an intervention (e.g. vasodilation agents, fluid challenges, or after a brain activation challenge), the empty frame may not be representative of the remainder of the scan. In these cases, correction factors may introduce significant errors.

There thus remains a need for improved non-invasive and efficient methods for using multi-scan PET diagnostics in clinical practice.

### SUMMARY OF THE INVENTION

The present invention relates to a non-invasive method for efficient PET diagnostics based on a radiopharmaceutical protocol which comprises repeated administration of a single radioisotope.

The inventors have found that instead of using a correction method based on an empty frame and subtracting that empty frame from the subsequent images, the standard pharmacokinetic model may be expanded with a residual signal parameter. Using this approach, residual correction is based on the data during the entire scan instead of only the start prior to the injection. As a result, the correction represents an average correction during the scan, which is expected to be more reliable. The result for a single-tissue model with blood spillover correction is as follows: ${C}_{tissue} \left(t\right) = {K}_{1} ⋅ {C}_{blood} \left(t\right) ⊗ {e}^{- {k}_{2} ⋅ t} + {V}_{blood} ⋅ {C}_{blood} \left(t\right) + RS$ in which RS stands for Residual Signal.

However, as the residual signal does not need to be assumed to be constant, the model can be extended to ${C}_{tissue} \left(t\right) = {K}_{1} ⋅ {C}_{blood} \left(t\right) ⊗ {e}^{- {k}_{2} ⋅ t} + {V}_{blood} ⋅ {C}_{blood} \left(t\right) + \left(RS+slope⋅t\right)$

According to the present invention the residual signal ***RS*** is not based on a single empty frame, but is estimated using all data that is used in the estimation of the pharmacokinetic parameters (K₁, K₂ and V_{blood} above).

The advantages of this are:
1. The method becomes less sensitive to errors between the empty frame and the remainder of the scan, or errors occurring during the scan. As the correction is based on the entire dataset, it is theoretically more stable.
2. The empty frame can be omitted, shortening protocol durations.
3. Corrections for residual activity can be expanded to include a slope.

Accordingly, in **a first aspect** of the invention there is provided a method for rapid serial, subtraction-free and non-invasive PET diagnostics in a human, which method comprises the following steps:
a) administering a radiopharmaceutical comprising a radioisotope with a half-life t_{half} to a human subject, followed by
b) optionally obtaining PET data of said human subject by performing a PET scan of the subject concurrently with or subsequent to introducing the first radiopharmaceutical, followed by
c) optionally moving said human subject in the scanner such that the organ or region of interest (ROI) for the second scan is in the scanner field of view, followed by
d) administering a **second dose of a radiopharmaceutical** comprising the same radioisotope as in step a, followed by
e) obtaining a series of sequential PET images of the ROI initiated prior to, or concurrently with introducing the second radiopharmaceutical, followed by
f) reconstructing the PET images for the second radiopharmaceutical using appropriate corrections for quantitative PET and attenuation into a multi-frame reconstruction, followed by
g) measuring the activity in the ROI and extracting the measured radioactivity concentrations at the various timepoints (time-activity curves, TACs), followed by
h) fitting the extracted TACs to a pharmacokinetic model which includes a correction parameter for residual activity of the first radiopharmaceutical, followed by
i) returning estimated (maximum-likelihood) parameter values of the pharmacokinetic model for the ROI including uptake rate, washout rate, and an estimate of the amount of residual activity of the first radiopharmaceutical,
wherein t_{half} is equal to or greater than 1/8^{h} of the time between the two dose administrations, and the two radiopharmaceuticals can be the same.

### BRIEF DESCRIPTION OF DRAWINGS

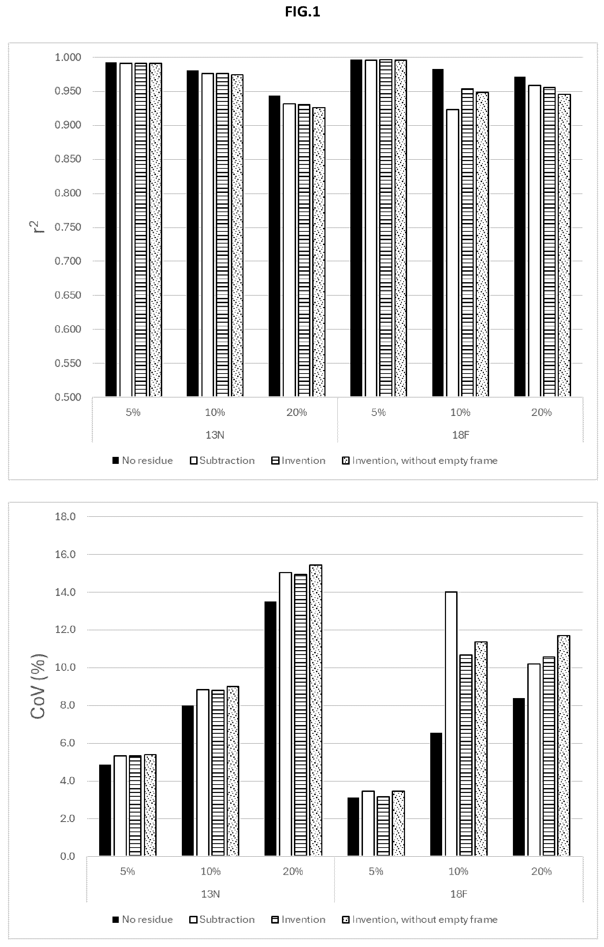

**Figures 1-3** shows the result of simulations performed in MATLAB. For each tracer (¹³N and ¹⁸F), a single set of 10.000 values for each parameter (K1, k2, VA, VV [optional], RS, and slope [optional]) was defined. Depending on the specific simulation, parameters were included or excluded. For each set of parameters, a true, noise-free time-activity curve (TAC) was defined, using a single randomly selected blood input function and a standard 10-minute scan following a 1-minute empty frame.
   Random Gaussian noise was predefined for each individual frame, multiplied with that frame's specific weight and the true TAC for that frame and simulation and added to the true TAC. This was done for both a curve with the residual signal and, as reference, one without residual signal. Finally, the noisy TACs were fed into a standard curve-fitting algorithm to get the least squares estimate of each parameter based on the noisy curve. This was performed for:
   1) The regular noisy TAC (striped bars),
   2) The noisy TAC where the empty frame was not included (dotted bars),
   3) The noisy TAC from which the activity of frame 1 was subtracted from all subsequent frames (empty bars), and
   4) The noisy TAC without residual signal (solid bars).
**Figure 1** In this setting, 5%, 10% and 20% noise was assessed for both ¹⁸F and ¹³N based tracers. No motion or slope was simulated. Results are given (Figure 1, top) for correlation (r²) and coefficient of variation (CoV, standard deviation / mean estimate * 100%, Figure 1, bottom).

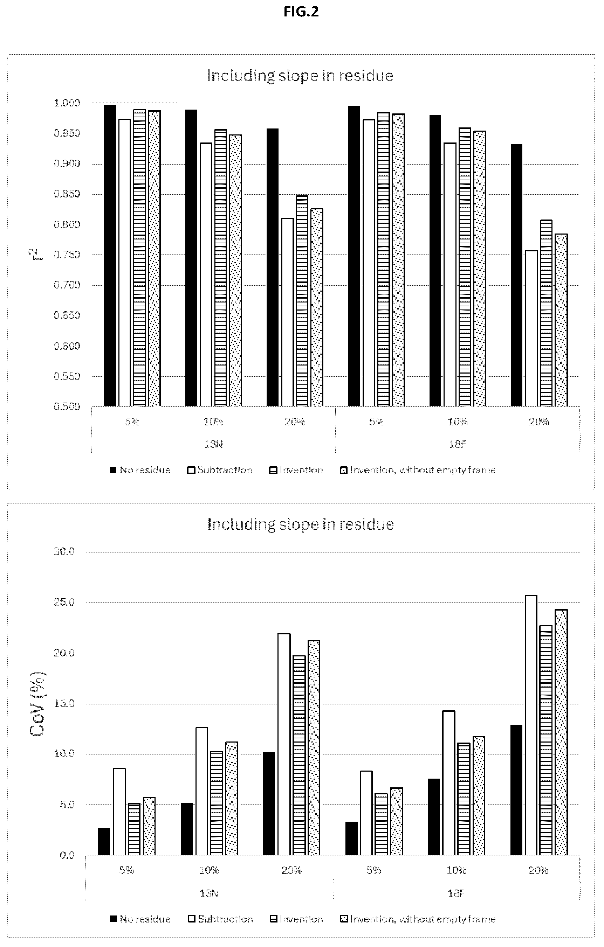

**Figure 2** In this setting, the method of the present invention outperformed the subtraction method as reflected by improved correlations and lower CoV values. This includes both the situation where the empty frame prior to injection of the second tracer is kept and the situation where this frame is not used.

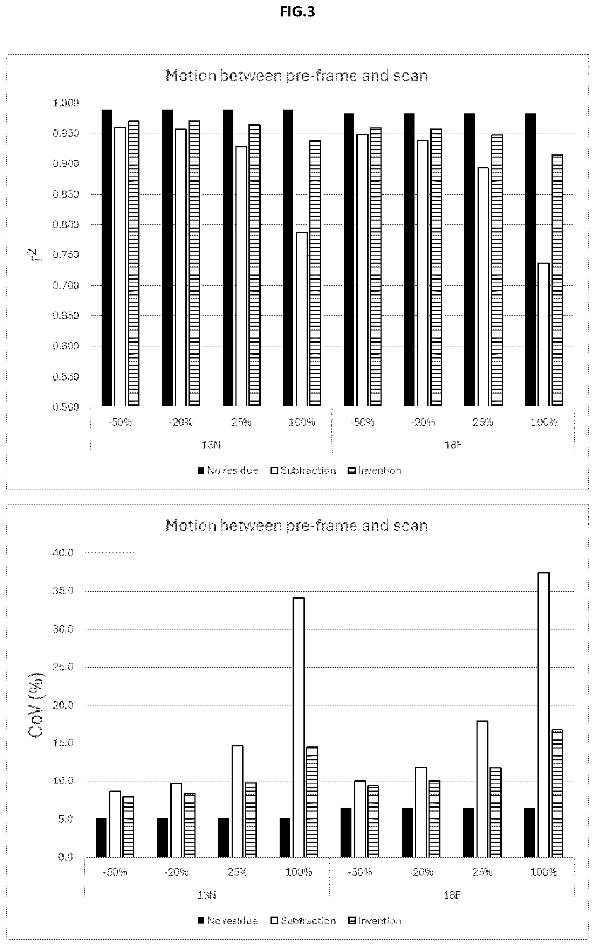

**Figure 3** In this setting, the subtraction method show significantly poorer performance as compared to the method of the present invention. When the initial activity was overestimated, results became unreliable with a CoV of 30% or more in the most severe cases. For the method of the present invention, variability was lower and more stable when motion increased in severity.

### DEFINITIONS

In the context of the present application the following definitions will be used:
PET = Positron emission tomography
TACs = Time-activity curves
ROI = region of interest
Radiopharmaceuticals = a group of pharmaceutical drugs containing radioactive isotopes.
¹³N-NH₃ or ¹³N-ammonia means nitrogen-13 labelled ammonia, which is a radioactive variation of regular ammonia in which the nitrogen atom has been replaced by nitrogen-13, which has a t_{1/2} of 10 minutes.
¹⁸F-flurpiridaz or Flurpiridaz (¹⁸F) is a cyclotron-produced radioactive diagnostic agent for use with positron emission tomography (PET) myocardial perfusion imaging under rest or stress. ¹⁸F has a t_{1/2} of 110 minutes.

### DETAILED DESCRIPTION

As described hereinabove, the present invention relates to a non-invasive method for efficient PET diagnostics based on a radiopharmaceutical protocol which comprises repeated administration of a radioisotope, which protocol provides a solution to the problems associated with prior art technology, making repeat scan PET diagnostics and, consequently, more personalized medicine feasible.

Repeat PET studies are of clinical benefit, specifically in coronary artery disease imaging where a rest scan is followed by a stress scan. For several radioisotopes, such as ¹⁵O (t_{1/2} = 122s) and ⁸²Rb (t_{1/2} = 76s), half-lives are sufficiently short that a brief pause in the clinical protocol allows for the second scan to be performed after the activity of the first has decayed to background levels.

For other tracers, such as ¹³N (t_{1/2} = 10 minutes) and ¹⁸F (t_{1/2} = 110 minutes), the longer half-lives render "waiting" protocols, which contain a pause between scans, prohibitively long. As a result, PET diagnostic multi-scan methods employing such longer-lived radioisotopes ( ¹³N and ¹⁸F ) become impractical.

To address this issue, a background correction method is commonly used in presently employed multi-scan methods employing ¹³N and ¹⁸F. Here, typically, the first injection is of a lower dose and the second injection is performed while there still is significant residual activity of the first injection present in the images. Prior to the second injection, the subject is imaged for 1-2 minutes. Then, the injection starts and the activity measured prior to injection ("empty frame") is subtracted from all successive frames.

This approach has *i.a*. been implemented in commercial software packages such as e.g. Invia's 4DM https://www.inviasolutions.com/packages), and can be represented as $\left({C}_{tissue}\left(t\right)-{C}_{tissue}\left({t}_{0}\right)\right) = {K}_{1} ⋅ {C}_{blood} \left(t\right) ⊗ {e}^{- {k}_{2} ⋅ t} + {V}_{blood} ⋅ {C}_{blood} \left(t\right)$

However, the subtraction method can be unstable, as it assumes that the empty frame is representative of the activity during the entire scan. This assumption is violated in cases of patient motion, when the patient is not under stable physiological conditions, or when the activity is not stable but increasing or decreasing. In these cases, correction factors may introduce significant errors.

The inventors have found that instead of using a correction method based on an empty frame and subtracting that empty frame from the subsequent images, the standard pharmacokinetic model may be expanded with a residual signal parameter, ***RS.*** According to the present invention, the residual signal ***RS*** is not based on a single empty frame but is estimated using all data that is used in the estimation of the pharmacokinetic parameters (K₁, K₂ and V_{blood} above).

The advantages of this approach are:
1. The method becomes less sensitive to errors between the empty frame and the remainder of the scan, or errors occurring during the scan. As the correction is based on the entire dataset, it is theoretically more stable.
2. The empty frame can be omitted, shortening protocol durations.
3. Corrections for residual activity can be expanded to include a slope.

In practice, the present invention entails the following steps:
1. Administer a first dose of a radiopharmaceutical with a radioisotope with a half-life t_{1/2}
2. Optionally image the patient for a certain amount of time in the PET scanner
   a. This can be a multi-frame acquisition or a single-frame acquisition
   b. This can be a single bed position or a multi-bed position acquisition
   c. This imaging can be any part of the body, and does not need to be the same part as for the next steps
3. Optionally (re)position the patient in the scanner such that the organ or region of interest (ROI) is in the scanner field of view,
4. Administer a second dose of a radiopharmaceutical comprising the same radioisotope as in step 1, followed by
5. Reconstruct the PET images using all corrections for the second radiopharmaceutical, using appropriate corrections for quantitative PET and attenuation into a multi-frame reconstruction, followed by
6. Measure the activity in the ROI and extract the measured radioactivity concentrations at the various timepoints (time-activity curves, TACs) , followed by
7. Fit the extracted TACs to a pharmacokinetic model which includes a correction parameter for residual activity of the first radiopharmaceutical, i.e. ${C}_{tissue} \left(t\right) = {K}_{1} ⋅ {C}_{blood} \left(t\right) ⊗ {e}^{- {k}_{2} ⋅ t} + {V}_{blood} ⋅ {C}_{blood} \left(t\right) + RS$ or, for situations where the residual signal is not assumed to be constant, the model can be extended to ${C}_{tissue} \left(t\right) = {K}_{1} ⋅ {C}_{blood} \left(t\right) ⊗ {e}^{- {k}_{2} ⋅ t} + {V}_{blood} ⋅ {C}_{blood} \left(t\right) + \left(RS+slope⋅t\right)$
in which **RS** stands for Residual Signal.

The result of step 7 is the standard parameters of the standard model (e.g. uptake rate, washout rate, blood flow) after correcting for the residual activity, and an estimate of the amount of residual activity of the first radioisotope. These values, or a subset of these, are returned for potential clinical interpretation. When the activity in step 7 is extracted for each pixel, images of the employed radiopharmaceutical's concentration can be extracted for evaluation.

Accordingly, in **a first aspect** of the invention there is provided a method for rapid serial, subtraction-free and non-invasive PET diagnostics in a human, which method comprises the following steps:
a) administering **a first dose** of a radiopharmaceutical comprising a radioisotope with a half-life t_{half} to a human subject, followed by
b) optionally obtaining PET data of said human subject by performing a PET scan of the subject concurrently with or subsequent to introducing the first radiopharmaceutical, followed by
c) optionally moving said human subject in the scanner such that the organ or region of interest (ROI) for the second scan is in the scanner field of view, followed by
d) administering **a second dose** of a radiopharmaceutical comprising the same radioisotope as in step a, followed by
e) obtaining a series of sequential PET images of the ROI initiated prior to, or concurrently with introducing the second radiopharmaceutical, followed by
f) reconstructing the PET images for the second radiopharmaceutical using appropriate corrections for quantitative PET and attenuation into a multi-frame reconstruction, followed by
g) measuring the activity in the ROI and extracting the measured radioactivity concentrations at the various timepoints (time-activity curves, TACs), followed by
h) fitting the extracted TACs to a pharmacokinetic model which includes a correction parameter for residual activity of the first radiopharmaceutical, followed by
i) returning estimated (maximum-likelihood) parameter values of the pharmacokinetic model for the ROI including uptake rate, washout rate, and an estimate of the amount of residual activity of the first radiopharmaceutical,
wherein t_{half} is equal to or greater than 1/8^{th} of the time between the two dose administrations.

The protocol according to the present invention enables the reliable acquisition and analysis of a second PET scan even if issues with patient motion occur between or during scans, or when the activity is not stable but increasing or decreasing. In particular, the protocol according to the present invention is more precise and robust than the traditional subtraction method in situations where the residual activity for some reason is gradually changing, or when the initial "background" image has been measured incorrectly.

Using this approach, a continuous correction is used, instead of a correction based on a single time-point, and the protocol can be shortened by eliminating the pre-scan delay typically used with the image subtraction method.

In an embodiment of the first aspect, the dose of the radiopharmaceutical in steps a) and d) is the same. In another embodiment of the first aspect, the dose of the radiopharmaceutical in steps a) is different from the dose of the radiopharmaceutical in step d).

In an embodiment of the first aspect, the two radiopharmaceuticals in steps a) and d) are the same. In another embodiment of the first aspect, the two radiopharmaceuticals in steps a) and d) are different, but comprise the same radioisotope.

The method of the present invention as described above is not restricted to the radioisotopes ¹³N and ¹⁸F, these serve as examples only. In an embodiment of the first aspect, the radioisotope comprised by the radiopharmaceutical in step a) and step d) is selected from ¹³N, ¹¹C, ¹⁸F, ⁶²Cu, ⁶⁴Cu, ⁶⁸Ga, ⁸⁹Zr and ¹²⁴I.

In a preferred embodiment of the first aspect, the radiopharmaceutical employed in step a) and d) is ¹³N-NH3. In another preferred embodiment of the first aspect, the radiopharmaceutical employed in step a) and d) is ¹⁸F-flurpiridaz. Radiopharmaceuticals based on the radioisotopes ⁶⁸Ga (t_{1/2} = 68 min) and/or ¹¹C (t_{1/2} = 20 min) are also well suited for being employed in the method of the present invention.

In an embodiment of the first aspect, for ¹³N-NH3, the time between injections in steps a) and d) will be around 20 minutes (20 minutes ±10 minutes). In another embodiment of the first aspect, for ¹⁸F-flurpiridaz, the time between injections in steps a) and d) will be around 20 minutes (20 minutes ±10 minutes).

In a preferred embodiment the first and second dose of radiopharmaceutical are administered as IV boluses.

In a preferred embodiment of any of the aspects of the present invention the method for subtraction-free and non-invasive PET diagnostics in a human is furthermore non-therapeutic.

In an embodiment the corrections for quantitative PET are selected from decay, scatter, dead-time or others as recommended by the PET scanner manufacturers. In another embodiment the multi-frame reconstruction comprises frames of 1x10, 8x5, 4x10, 2x15, 3x20, 2x30, 2x60 seconds duration, for a total duration of about 6 minutes.

In another embodiment, **RS or RS** + **slope*t** is added to an irreversible or reversible two-tissue pharmacokinetic models.

The present disclosure further relates to a method for rapid multi-scan imaging, which may be used clinically for e.g. assessing coronary artery disease by comparing resting and pharmacological vasodilation myocardial blood flow, or cerebral blood flow at baseline and after a stimulus is provided.

### EXPERIMENTAL

The method of the present invention was validated using simulated data, for two different tracers, ¹³N-NH₃ and ¹⁸F-flurpiridaz.

Using MATLAB, and for each tracer, a single set of 10.000 values for each parameter (K1, k2, VA, VV [optional], RS, and slope [optional]) was defined. Depending on the specific simulation, parameters were included or excluded. For each set of parameters, a true, noise-free time-activity curve (TAC) was defined, using a randomly selected blood input function and a standard 10-minute scan following a 1-minute empty frame. As these TACs are entirely simulated and noise-free, we can create a TAC with the residual signal and one without, and directly compare obtained values.

Random Gaussian noise was predefined for each individual frame, multiplied with that frame's specific weight and the true TAC for that frame and simulation and added to the true TAC. While mean noise levels were equal between tracers, noise levels varied between frames based on the radioisotope's half-life and the frame duration.

This was done for both a curve with the residual signal and, as reference, one without residual signal. Finally, the noisy TACs were fed into a standard curve-fitting algorithm to get the least squares estimate of each parameter based on the noisy curve. This was performed for:
1) The regular noisy TAC (striped),
2) The noisy TAC where the empty frame was not included (dotted),
3) The noisy TAC from which the activity of frame 1 was subtracted from all subsequent frames (empty bar), and
4) The noisy TAC without residual signal (solid bar).

All obtained values were then compared to the values used to generate the noise-free TACs for correlation and variability.

Several simulation settings were tested:
A. Unless otherwise stated, a 10% noise level was assumed, but a single set of simulations was performed at 5% or 20% noise.
B. Simulations were performed both with and without inclusion of the slope in the true TACs
C. To simulate errors/motion, sets of simulations were performed where the activity in the empty frame (only) was multiplied with a factor of 0.50, 0.80, 1.25 or 2.00. This simulations motion of an adjacent organ with lower residual activity into the region of interest (factor > 1) or motion of a region with high activity out of the region of interest (factor <1)

### Simulations

In the standard setting, 5%, 10% and 20% noise was assessed for both ¹⁸F and ¹³N based tracers. No motion or slope was simulated. Results can be seen in **Figure 1** (top and bottom) for correlation (r²) and coefficient of variation (CoV, i.e. standard deviation / mean estimate * 100%).

As can be seen from **Figure 1** (top and bottom), in the normal case, all methods perform approximately comparable in correcting for the residual activity. This includes the method where the initial empty frame is discarded, allowing for short scanning protocols. All three correction methods did perform slightly worse than the case where no residual signal was present at all.

When the residual signal was assumed to not be constant, but linearly increasing or decreasing, the results of the simulation were as presented in Figure 2 (top and bottom). As can be seen from Figure 2, the invention outperformed the subtraction method as reflected by improved correlations (r²) and lower CoV values. This includes both the situation where an empty frame prior to injection of the second tracer is kept and the situation where this frame is not used.

In the final set of simulations, an error in the measured value of the initial frame was simulated **(****Figure 3****,** top and bottom). This resembles motion between the empty frame and the remainder of the scan, causing a region with high activity to be moved out of the region of interest, or an adjacent region with higher activity to be moved into the region of interest. Note that the setting where the empty frame was not measured is not included in this set of simulations, as all values would by definition be identical when motion in the excluded empty frame was simulated. The values in the horizontal axis represent the factor by which the activity in the initial empty frame is multiplied.

As can be seen from **Figure 3** (top and bottom), the subtraction method show significantly poorer performance as compared to the method according to the present invention. When the initial activity was overestimated, results became unreliable with a CoV of 30% or more in the most severe cases. For the method according to the invention, variability was lower and more stable when motion increased in severity.

## Claims

1. A method for serial, subtraction-free and non-invasive PET diagnostics in a human, which method comprises the following steps:
a) administering **a first dose** of a radiopharmaceutical comprising a radioisotope with a half-life t_{half} to a human subject, followed by
b) optionally obtaining PET data of said human subject by performing a PET scan of the subject concurrently with or subsequent to introducing the first radiopharmaceutical, followed by
c) optionally moving said human subject in the scanner such that the organ or region of interest (ROI) for the second scan is in the scanner field of view, followed by
d) administering **a second dose** of a radiopharmaceutical comprising the same radioisotope as in step a, followed by
e) obtaining a series of sequential PET images of the ROI initiated prior to, or concurrently with introducing the second radiopharmaceutical, followed by
f) reconstructing the PET images for the second radiopharmaceutical using appropriate corrections for quantitative PET and attenuation into a multi-frame reconstruction, followed by
g) measuring the activity in the ROI and extracting the measured radioactivity concentrations at the various timepoints (time-activity curves, TACs), followed by
h) fitting the extracted TACs to a pharmacokinetic model which includes a correction parameter for residual activity of the first radiopharmaceutical, followed by
i) returning estimated (maximum-likelihood) parameter values of the pharmacokinetic model for the ROI including uptake rate, washout rate, and an estimate of the amount of residual activity of the first radiopharmaceutical,
wherein t_{half} is equal to or greater than 1/8^{th} of the time between the two dose administrations.

2. The method according to any one of claim 1, wherein the radiopharmaceutical contains a radioisotope selected from ¹³N, ¹¹C, ¹⁸F, ⁶²Cu, ⁶⁴Cu, ⁶⁸Ga, ⁸⁹Zr and ¹²⁴I.

3. The method according to any one of claim 1 or claim 2, wherein the radiopharmaceutical contains a radioisotope selected from selected from ¹³N and ¹⁸F.

4. The method according to any one of claim 1 -3, wherein the radiopharmaceutical contains ¹⁸F.

5. The method according to any one of claim 1 - 4, wherein the radiopharmaceutical is ¹⁸F- flurpiridaz.

6. The method according to any one of claim 1 - 3, wherein the radiopharmaceutical contains ¹³N.

7. The method according to any one of claim 1 - 3 or 6, wherein the radiopharmaceutical is ¹³N-NH₃ (¹³N-ammonia).

8. The method according to any one of claim 1 - 7 wherein both the first and second doses of radiopharmaceutical are administered as IV boluses.

9. The method according to any one of claim 1 - 8 wherein the extracted TACs are fitted to a pharmacokinetic model: ${C}_{tissue} \left(t\right) = {K}_{1} ⋅ {C}_{blood} \left(t\right) ⊗ {e}^{- {k}_{2} ⋅ t} + {V}_{blood} ⋅ {C}_{blood} \left(t\right) + RS$ in which Cₜᵢₛₛᵤₑ₍ₜ₎ and C_{blood(t)} are the time-activity curves extracted in step g, **K₁** and **k₂** are the uptake and washout rates of the second administered dose radiopharmaceutical and represent blood flow, V_{blood} is a spillover correction and ***RS*** stands for Residual Signal of the first administered dose of radioisotope.

10. The method according to claim 10, wherein the estimated constant residual activity ***RS*** of the first administered dose of radioisotope is replaced by a linear function **(*****RS** + **slope*** · *t*), where ***slope*** · *t* represents the linear increase or decrease in the activity of ***RS,** i.e.:* ${C}_{tissue} \left(t\right) = {K}_{1} ⋅ {C}_{blood} \left(t\right) ⊗ {e}^{- {k}_{2} ⋅ t} + {V}_{blood} ⋅ {C}_{blood} \left(t\right) + \left(RS+slope⋅t\right)$

11. The method according to any one of claim 1- 10 **characterized in** being non-therapeutic.

12. The method according to any one of claim 1 - 11 wherein the returned estimated (maximum-likelihood) parameter values of the pharmacokinetic model for the ROI, which include blood flow and an estimate of the amount of residual activity of the first radiopharmaceutical in the ROI are used in clinical diagnosis.
